(19) Europäisches Patentamt
European Patent Office
Office européen des brevets

(11) **EP 4 553 497 B1**

(12) **EUROPÄISCHE PATENTSCHRIFT**

(45) Veröffentlichungstag und Bekanntmachung des Hinweises auf die Patenterteilung:
**29.04.2026  Patentblatt 2026/18**

(21) Anmeldenummer: **23208547.2**

(22) Anmeldetag: **08.11.2023**

(51) Internationale Patentklassifikation (IPC):
**G01N 33/04** (2006.01)    **G06Q 50/04** (2012.01)

(52) Gemeinsame Patentklassifikation (CPC):
**G01N 33/04; G06Q 10/04; G06Q 50/02; G06Q 50/04**

(54) **VERFAHREN ZUR VORHERSAGE DER ROHSTOFFFUNKTIONALITÄT IM ENDPRODUKT**

METHOD FOR PREDICTING THE RAW MATERIAL FUNCTIONALITY IN THE END PRODUCT

PROCÉDURE DE PRÉDICTION DE LA FONCTIONNALITÉ DES MATIÈRES PREMIÈRES DANS LE PRODUIT FINAL

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(43) Veröffentlichungstag der Anmeldung:
**14.05.2025  Patentblatt 2025/20**

(73) Patentinhaber: **DMK Deutsches Milchkontor GmbH**
**27404 Zeven (DE)**

(72) Erfinder:
• **ZINK, Ralf**
**26160 Bad Zwischenahn (DE)**
• **BODE, Katja**
**21279 Hollenstedt (DE)**
• **SCHULZ, Maren Christina**
**27412 Wilstedt (DE)**

(74) Vertreter: **Fabry, Bernd**
**IP2 Patentanwalts GmbH**
**Schlossstrasse 523-525**
**41238 Mönchengladbach (DE)**

(56) Entgegenhaltungen:
**JP-A- 2022 121 218**

• **MAKHOUL SALIM ET AL: "Rapid non-invasive quality control of semi-finished products for the food industry by direct injection mass spectrometry headspace analysis: the case of milk powder, whey powder and anhydrous milk fat", JOURNAL OF MASS SPECTROMETRY, vol. 51, no. 9, 30 August 2016 (2016-08-30), pages 782 - 791, XP093141973, ISSN: 1076-5174, DOI: 10.1002/jms.3801**
• **PASCUAL LLUIS ET AL: "A voltammetric e-tongue tool for the emulation of the sensorial analysis and the discrimination of vegetal milks", SENSORS AND ACTUATORS B: CHEMICAL, vol. 270, 4 May 2018 (2018-05-04), pages 231 - 238, XP093141167, ISSN: 0925-4005, DOI: 10.1016/j.snb.2018.04.151**

**Beschreibung**

**GEBIET DER ERFINDUNG**

Die vorliegende Erfindung befindet sich auf dem Gebiet der Lebensmitteltechnologie und betrifft ein Verfahren zur Vorhersage der Rohstofffunktionalität in Endprodukten insbesondere in Milchprodukten, Produkten auf primärer und hauptsächlicher Basis von Milch inklusive von Käsen deren pflanzlichen Milchalternativen sowie entsprechenden Hybridprodukten unter Erhebung definierter Analyse- und Rohstoffdaten und deren statistischer Auswertung.

**TECHNOLOGISCHER HINTERGRUND**

In Deutschland ist der Trend der veganen Ernährung angekommen. Es ernähren sich rund 1,3 Millionen Menschen vegan. Im Vergleich dazu ernähren sich circa 8 Millionen vegetarisch. Doch die Zahlen steigen täglich. Damit steigt auch das Interesse an pflanzlichen Rohstoffen, die geeignet sind, tierische Produkte zu ersetzen. Im Bereich der Milchprodukte wird dabei u. a. nach Ersatzstoffen für die wichtigen Milchproteine gesucht. Kandidaten dafür sind beispielsweise pflanzliche Proteine, wie sie beispielsweise aus der Erbse, der Ackerbohne oder dem Hafer gewonnen werden können. Problematisch ist jedoch zum einem der folgende Aspekt: Milchproteine können nicht 1:1 gegen beliebige andere Proteintypen ausgetauscht werden, weil zum einen die Rezepturen auf Milch als Proteinquelle ausgerichtet sind und zum anderen die Proteine durchaus sehr unterschiedliche Strukturen und Funktionalitäten aufweisen können. Gleiches gilt für den Austausch tierischer und/oder pflanzlicher Makronährstoffe wie Fett und Kohlenhydrate. Ein weiterer Aspekt der sich auf die Rohstofffunktionalität im Endprodukt auswirken kann, besteht darin, dass die Rohstoffherstellungsprozesse lieferantenabhängig variieren und hieraus unterschiedliche Rohstoffqualitäten und Chargen resultieren. Im Ergebnis weisen dann bei gewichtsgleichem Rohstoffaustausch die resultierenden Produkte beispielsweise auf einmal statt einer strukturierten Textur eine mehlige, gummiartige oder stumpfe Textur auf.

Um dem entgegenzuwirken ist es bisher erforderlich, potenziell geeignete Kandidaten zunächst in verschiedene Endprodukte einzuarbeiten und die gewünschten Eigenschaften, die sich dabei einstellen, zu überprüfen. Angesichts der Tatsache, dass der Markt inzwischen hunderte unterschiedliche Rohstoffe und Zusatzstoffe zur Beeinflussung der Endproduktzusammensetzung-, struktur und -stabilität sowohl auf Milch- als auch pflanzlicher Basis anbietet, ist die erforderliche Vorauswahl äußerst zeitaufwendig. Wünschenswert wäre daher ein Verfahren, das eine rasche Vorhersage der zu erwartenden Eigenschaften in den Endprodukten erlaubt.

**STAND DER TECHNIK**

Aus dem Stand der Technik sind Bewertungsverfahren bekannt, mit deren Hilfe man beispielsweise die Haltbarkeit von Lebensmitteln in einer Verpackung vorhersagen kann. So wird beispielsweise in der EP 1626275 B1 (WILD) ein Verfahren zur Bewertung der Produkthaltbarkeit in einem Packmittel vorgeschlagen, das die folgenden Schritte umfasst:

a) Einbringen eines mit Füllgut gefüllten, verschlossenen Packmittels (4) in eine Überdruckkammer (1),

b) Lagern des Packmittels (4) in der Überdruckkammer (1) über einen Zeitraum $t_1$ bei Überdruck $p_1$ und einer bestimmten Temperatur $T_1$ in einer Testgasatmosphäre, wodurch eine definierte Menge Q an Testgas in das Packmittel permeiert,

c) Lagern des Packmittels (4) über einen bestimmten Zeitraum $t_2$ unter Wärme und/oder Lichteinfluss, und

d) analytische und/oder sensorische Untersuchung des Füllguts.

Nachteilig bei dem Verfahren ist der hohe Apparateaufwand, zudem lässt das Funktionsprinzip - Nachbildung der in das Füllgut eingebrachten Sauerstoffmenge und Beschleunigung der Reaktion durch erhöhten Druck- letztlich nur eine grobe Schätzung zu, die für eine qualitative Aussage vielfach ungeeignet ist, zumal bekannt ist, dass für viele Polymer/Gas-Systeme Löslichkeits- und Diffusionskoeffizient der permeierenden Substanz im Polymer (z.B. einer Plastikflasche) von Konzentration und Druck abhängig werden, wenn die Untersuchung unterhalb der Glastemperatur Tg der Polymere und/oder der kritischen Temperatur des Gases durchgeführt werden [MÜLLER, K. "Sauerstoff-Durchlässigkeit von Kunststoffflaschen und Verschlüssen" Dissertation TU München (2003)]

Eine völlig andere Methode, die Haltbarkeit eines verpackten Lebensmittels für den Verbraucher optisch anzuzeigen, ist Gegenstand der Schutzrechte EP 2378354 B1, EP 2581785 B1 sowie WO 2009 056591 A1 (UNI MÜNSTER). Diese betreffen eine Sensorvorrichtung, konkret einen elektrochemischen Prozessor, der mit dem Öffnen der Verpackung kurzgeschlossen wird. Es bildet sich eine Aluminiumoxidschicht, deren Fortschreiten proportional zur Haltbarkeit des Produktes verläuft. Eine Temperaturerhöhung führt beispielsweise dazu, dass die Oxidbildung beschleunigt wird und spiegelt damit natürlich auch den abnehmenden Frischegrad des Produktes wider. Diese Lösung macht es indes erforderlich, jede einzelne Verpackung mit einem separaten Sensor auszustatten, was ebenfalls technisch aufwendig ist.

EP 3875953 B1 (DMK) beschreibt einen beschleunigten Lagertest zur Bestimmung von Qualität und Haltbarkeit von Lebensmitteln, bei dem eine erste Probe bei einer Temperatur T1 und eine gleichartige zweite Probe bei einer deutlich höheren Temperatur T2 gelagert werden, wobei man die Proben sensorisch beurteilt und über eine Kalibrierkurve miteinander verglichen werden. Da diese sensorische Beurteilung von Produkten stets eine subjektive Komponente enthält, wäre eine Methode, die ausschließlich auf objektiven Parametern beruht vorteilhafter.

Keines dieser bestehenden Untersuchungsverfahren beschäftigt sich mit der frühzeitigen, gezielten Vorhersage der Rohstofffunktionalität im Endprodukt.

Ferner offenbart JP2022121218 ein Verfahren zur Vorhersage der Rohstofffunktionalität in Butter, mit den Schritten: Bereitstellen von Rohmilchproben unterschiedlicher Qualitäten; Herstellung von Butter aus diesen Rohmilchproben; Erfassung und Bewertung der Produkteigenschaften; Analyse der Daten mit Hauptkomponentenanalyse unter Erhalt eines Score-Plots; Zuordnung der Rohstoffqualitäten zu den Datenpunkten im Score-Plot; und Ermitteln von Clustern im Score-Plot, zur Erkennung welche Rohstoffe zu ähnlichen oder gleichen Produkteigenschaften der Butter führen.

## AUFGABE DER ERFINDUNG

Daher hat die Aufgabe der vorliegenden Erfindung darin bestanden, ein Testverfahren bereitzustellen, welches durch die korrelierte Betrachtung und statistische Auswertung diverser Rohstoffeigenschaften erlaubt, die Rohstofffunktionalität in Endprodukten wie Milchprodukten, Produkten auf primärer und hauptsächlicher Basis von Milch inklusive von Käsen deren pflanzlichen Milchalternativen sowie entsprechenden Hybridprodukten zuverlässig frühzeitig und reproduzierbar vorherzusagen.

## BESCHREIBUNG DER ERFINDUNG

Die Erfindung betrifft ein Verfahren zur Vorhersage der Rohstofffunktionalität in Endprodukten wie Milchprodukten, Produkten auf primärer und hauptsächlicher Basis von Milch inklusive von Käsen deren pflanzlichen Milchalternativen sowie entsprechenden Hybridprodukten, umfassend oder bestehend aus den folgenden Schritten

(a) Bereitstellen eines Satzes von Rohstoffen unterschiedlicher Qualitäten;
(b) Erstellen eines Sets von Prüfparametern;
(c) Anwendung der festgelegten Prüfparameter auf jede der einzelnen Proben aus Schritt (a) unter Erzeugung eines Datensatzes;
(d) Einarbeiten der Rohstoffe unterschiedlicher Qualitäten aus Schritt (a) in Endprodukte wie Milchprodukte, Produkte auf primärer und hauptsächlicher Basis von Milch inklusive von Käsen deren pflanzlichen Milchalternativen sowie entsprechenden Hybridprodukten;
(e) Erfassung und Bewertung der Produkteigenschaften;
(f) Überprüfung des Zusammenhangs der Daten im Datensatz mit Hilfe der Principal Component Analysis (PCA) unter Erhalt eines Koordinatensystems (Score-Plot) und Ermittlung der größten Varianz der Daten als erster Komponente und der zweitgrößten Varianz der Daten als zweiter Komponente;
(g) Zuordnung der gefundenen Produkteigenschaften aus Schritt (e) zu den Datenpunkten im Koordinatensystem aus Schritt (f); und
(h) Ermitteln von Clustern im Score-Plot zur Identifikation von Rohstoffen, die beim Einsatz im Endprodukt zu den gewünschten ähnlichen oder gleichen Produkteigenschaften führen.

Überraschenderweise wurde gefunden, dass sich mit Hilfe des erfindungsgemäßen Verfahrens neue Rohstoffe hinsichtlich unterschiedlicher Eigenschaften und Funktionalitäten, die sie bei der Einarbeitung in unterschiedlichen Milchprodukten, Produkten auf primärer und hauptsächlicher Basis von Milch inklusive von Käsen deren pflanzlichen Milchalternativen sowie entsprechenden Hybridprodukten hervorrufen, vorab auf der Grundlage von physikochemischen Eigenschaften schnell, sicher und reproduzierbar beurteilen lassen, die bei der Eingangsprüfung der Rohstoffe überwiegend ohnehin erfasst, aber nicht für eine beschriebene Vorhersage verwendet werden.

Das erfindungsgemäße Verfahren gliedert sich in vier Abschnitte:

- Auswahl geeigneter Analyseparameter

- Datengenerierung

- Überprüfung des Zusammenhangs der Daten durch PCA sowie

- Ermitteln von Clustern im Score-Plot zur Identifikation von Rohstoffen, die beim Einsatz im Produkt zu der gewünschten ähnlichen oder gleicher Produkteigenschaften führen.

**Milchprodukte, Produkte auf primärer und hauptsächlicher Basis von Milch inklusive von Käsen oder deren pflanzlicher Milchalternativen und entsprechenden Hybridprodukten**

Die Auswahl der Produkte, auf die das erfindungsgemäße Verfahren angewendet werden kann, ist an sich unkritisch. Typischerweise sind die Produkte ausgewählt aus der Gruppe, die gebildet wird von Vollmilch, Magermilch, UHT-Milch, Milchpulvern, Sahne, Quark, Käse und Joghurt, aber auch Produkte auf primärer und hauptsächlicher Basis von Milch, wie beispielsweise zahlreiche Süßspeisen. Ebenfalls umfasst sind deren pflanzlichen Milchalternativen wie u. a. pflanzliche Drinks, Spreads, Sahnealternativen, Joghurtalternativen und Käsealternativen. Ebenfalls kann das Verfahren auf Hybridprodukte angewendet werden, bei denen nur ein Teil der tierischen Milchbestandteile gegen pflanzliche Alternativen ausgetauscht worden sind oder umgekehrt (Austausch pflanzlicher Komponenten gegen tierische Rohstoffe bzw. Inhaltsstoffe). Diese Aufzählung ist nicht als abschließend anzusehen.

**Rohstoffe**

Die Rohstoffe, deren Eigenschaften und Funktionalitäten in den Endprodukten vorhergesagt werden sollen, können ausgewählt sein aus der Gruppe, die gebildet wird von Rohstoffen wie Proteinen, Fetten, Kohlenhydraten, aber auch Halbfabrikaten wie Sauermolke, Milchpermeate und Milchretentate, deren Mischungen sowie deren entsprechenden pflanzlichen Ersatzstoffen bzw. Rohstoffe. Sie können in fester, pulvriger Form oder auch flüssig und konzentriert vorliegen.

Üblicherweise wird für die angestrebte Vorhersage der Rohstofffunktionalität im Endprodukt ein Rohstoffsatz von 5 bis 50, vorzugsweise 7 bis 15 Rohstoffen benötigt, der vorzugsweise zu mindestens drei unterschiedlichen Endprodukteigenschaften führt.

**Prüfparameter**

Ein wesentliches Merkmal des erfindungsgemäßen Verfahrens ist die Auswahl der Prüfparameter auf deren Grundlage die Bewertung der Proben erfolgen soll. In Betracht kommen dafür vorzugsweise die Folgenden:

- pH-Wert;

- Löslichkeit;

- thermisches Verhalten (DSC);

- Partikelgrößenverteilung (D10, D50, D90, spezifische Oberfläche);

- Fettaufnahmevermögen (Rapsöl);

- Wasseraufnahmevermögen;

- gelöste Sauerstoffmenge;

- Instabilitätsindex (Lumisizer);

- Helligkeitsfarbwert weiß/schwarz (L*);

- Farbkoordinate rot/grün (a*);

- Farbkoordinate gelb/blau (b*);

- freier Aminostickstoff (PAN);

- Ammoniakgehalt;

- Harnstoffgehalt;

- Calciumgehalt;

- Säuregehalt;

- rheologisches Verhalten;

- Festigkeit; sowie

- Syneräsegrad.

Bei den DSC-Daten handelt es sich um die Ergebnisse, die bei der Differentialthermoanalyse gewonnen werden. Dabei handelt es sich um ein Verfahren der thermischen Analyse zur Messung von abgegebener oder aufgenommener Wärmemenge einer Probe bei Aufheizung, Abkühlung oder einem isothermen Prozess. Dabei wird ein verkapselter Behälter mit einer Probe (5-40 mg) und ein zweiter gleicher Behälter ohne Inhalt (Referenz) oder einer Referenzprobe gemeinsam in einer Temperaturkammer dem gleichen Temperaturänderungsprogramm ausgesetzt. Dabei kommt es infolge der Wärmekapazität der Probe und etwaigen exothermen oder endothermen Prozessen bzw. Phasenänderungen wie Schmelzen oder Verdampfen zu Temperaturdifferenzen zwischen Probe und Referenz, da bei dem untersuchten Prozess thermische Energie in die oder aus der Probe fließt. Die DSC dient vor allem der Feststellung des Kristallisationsgrades, des Schmelz- bzw. Zersetzungspunktes sowie des Proteindenaturierungsgrades. Gemessen werden u. a. die Temperatur, bei der die thermisch induzierte Reaktion beginnt (Onset) und endet (Endset), die Temperatur bei der die Reaktion ihr Maximum erreicht (Peak) und die Höhe des Peaks. Ebenfalls bestimmt wird dabei die Reaktionsenthalpie.

Die spezifische Oberfläche und die Partikelgrößenverteilung der Pulver wurde mittels statischer Laserlichtstreuung bestimmt (Horiba LA-960). Die spezifische Oberfläche der Pulver ist im Sinne des erfindungsgemäßen Verfahrens eine volumenbezogene Größe $S_v$ mit der Einheit $m^2/m^3$. Bestimmt wird dabei in der Regel die massenbezogene spezifische Oberfläche $S_M$, da dies experimentell leichter durchzuführen ist. Die beiden Werte lassen sich durch den Faktor $\rho$ umrechnen:

$$S_V = \rho\, S_M$$

Die Partikelgrößenverteilung umfasst die Angabe der D10, D50 und D90-Werte, d. h. die Angabe der Partikelgrößenbereiche, die 10, 50 oder 90 Gew.-% aller Pulverteilchen umfassen.

Das Fettaufnahmevermögen (hier das Aufnahmevermögen von Rapsöl) sowie das Wasseraufnahmevermögen betreffen die Quellfähigkeit der Pulver und werden in g Fett bzw. g Wasser pro g Pulver angegeben.

Der Helligkeitsfarbwert L* sowie die beiden Farbkoordinaten a* und b* gehören zum sogenannten L*a*b* Farbsystem, das auch als CIELAB-System bezeichnet wird. Es handelt sich um das heute gebräuchlichste System für die Farbmessung und hat in fast allen Anwendungsbereichen eine große Verbreitung gefunden. Es wurde 1976 als einer der gleichabständigen Farbräume von der CIE definiert, um dem Hauptproblem des ursprünglichen Yxy-Systems zu begegnen: Gleiche geometrische Differenzstrecken im x,y-Farbdreieck führen nicht zu empfindungsgemäß gleichen Farbunterschieden. Der Farbraum des L*a*b*-Systems ist durch die Helligkeit L* und die Farbkoordinaten a* und b* gekennzeichnet. Die Vorzeichen lassen die Farbrichtung erkennen: +a* deutet auf einen Rotanteil hin, während -a* in Richtung Grün zeigt. Dementsprechend steht +b* für Gelb, und -b* für Blau. Im Koordinatenursprung (Achsenschnittpunkt) befindet sich ein neutrales Grau ohne jede Buntheit. Mit wachsenden a*b*-Werten, je weiter also der Farbort von der Mitte entfernt liegt, wird die Buntheit größer. Für die Ermittlung der Farbwerte hat sich im Rahmen des erfindungsgemäßen Verfahrens das Gerät CR 400 bzw. 410 bewährt. Als weiterführende Informationen finden sich in der Verbraucherinformation "Exakte Farbkommunikation" der Firma Konica Minolta.

Vorzugsweise sollte das Set von Prüfparametern mindestens 5, vorzugsweise mindestens 7 Parameter enthalten.

**Principal Component Analysis (PCA)**

Der so erhaltene Datenpool wird im nächsten Schritt mit Hilfe der sogenannten "Principal Component Analysis" (PCA), die auch als Hauptkomponenten-Analyse bezeichnet wird, ausgewertet.

Dabei handelt es sich um ein etabliertes statistisches Verfahren zur Analyse großer Datensätze mit einer hohen Anzahl von Parametern pro Beobachtung, zur Verbesserung der Interpretierbarkeit von Daten unter Beibehaltung eines Höchstmaßes an Informationen und zur Visualisierung mehrdimensionaler Daten. Formal gesehen ist die PCA eine statistische Technik zur Reduzierung der Dimensionalität eines Datensatzes. Dies geschieht durch eine lineare Transformation der Daten in ein neues Koordinatensystem, in dem (der größte Teil) der Variation in den Daten mit weniger Dimensionen beschrieben werden kann als in den Ausgangsdaten. Im Rahmen des erfindungsgemäßen Verfahrens werden nur die ersten beiden Hauptkomponenten verwendet, um die Daten in zwei Dimensionen darzustellen und visuell Cluster von eng zusammenhängenden Datenpunkten zu identifizieren.

Bei der PCA-Datenanalyse wird die erste Hauptkomponente eines Satzes von p Variablen, von denen angenommen wird, dass sie gemeinsam normal verteilt sind, die abgeleitete Variable, die als lineare Kombination der ursprünglichen Variablen gebildet wird und die größte Varianz erklärt. Die zweite Hauptkomponente erklärt die größte Varianz in dem, was übrig bleibt, wenn der Effekt der ersten Komponente entfernt wird, und wir können fortfahren mit p Iterationen, bis die gesamte Varianz erklärt ist. Die PCA wird am häufigsten verwendet, wenn viele der Variablen stark miteinander korreliert sind und es wünschenswert ist, ihre Anzahl auf einen unabhängigen Satz zu reduzieren. Die erste bzw. zweite Hauptkomponente kann entsprechend als eine Richtung definiert werden, die die Varianz der projizierten Daten maximiert. Die PCA ist die einfachste der echten eigenvektorbasierten multivariaten Analysen und ist eng mit der Faktorenanalyse verwandt. Eine ausführliche Darstellung der Methode findet sich beispielsweise in

https://en.wikipedia.orgi/wiki/Principal component analysis

Kurz zusammengefasst liefert die PCA ein Koordinatensystem, bei dem für jeden Datenpunkt die Komponente mit der größten Varianz oder Abweichung ("Erste Komponente") gegen die Komponente mit der zweitgrößten Varianz ("Zweite Komponente) aufgetragen ist. Jedem der in dem Koordinatensystem befindlichen Datenpunkte wird nun im letzten Schritt die jeweilige zuvor bestimmte Produkteigenschaft zugeordnet.

Überraschenderweise wurde dabei gefunden, dass eine Clusterbildung resultiert, die mit zu untersuchenden Eigenschaften und Funktionalitäten eindeutig korreliert werden können. Zu den Eigenschaften und Funktionalitäten, die auf diese Weise untersucht bzw. vorhergesagt werden können, gehören beispielsweise Textur, Cremigkeit, Synärese, Mundgefühl, Festigkeit, Emulsionsstabilität, Synergieeffekte sowie Kombinationen von zwei, drei oder mehreren dieser Eigenschaften.

Dies bedeutet, dass eine unbekannte Rohstoffprobe lediglich auf die eingangs genannten Prüfparameter analysiert werden muss, um ihr dann mit Hilfe der PCA eine Koordinate zuordnen zu können. Aufgrund der Lage der Koordinate kann dann auf die voraussichtliche Rohstofffunktionalität in der Endformulierung schnell, sicher und ggf. automatisiert geschlossen werden. Eine noch schnellere Simulation aufgrund der Ausgangswerte und zukünftiger Vorhersage- und Selektionskriterien zur Rohstoffauswahl durch KI (künstliche Intelligenz) in einem noch beschleunigteren Ansatz ist daher einfach als zweite Stufe denkbar und umsetzbar. Weiterhin ist eine frühzeitige und schnelle Auswahl von geeigneten Alternativ-Rohstoffen in jeglicher industrieller Umsetzung möglich.

**Beispiel 1A/B**

*Parameterauswahl und Datengenerierung*

Nachfolgend wird das erfindungsgemäße Verfahren an Hand der Beurteilung verschiedener pflanzenbasierter Pulver erläutert. Dazu wurde zunächst die Proteinfraktion aus den Früchten verschiedener Pflanzen (Erbse, Ackerbohne, Kichererbse, Hafer) extrahiert und anschließend in einen pulverförmigen Zustand gebracht. Dann wurden aus den zur Verfügung stehenden Parametern die folgenden ausgewählt:

- pH-Wert
- Löslichkeit
- DSC-Daten (Onset, Endset, Peak, Peakhöhe, Reaktionsenthalpie)
- Partikelgrößenverteilung (D10, D50, D90, spezifische Oberfläche)
- Fettaufnahmevermögen (Rapsöl)
- Wasseraufnahmevermögen
- Farbkoordinaten (L*, a*, b*)

und die entsprechenden Messwerte bestimmt; diese sind in **Tabelle 1** zusammengefasst.
Anschließend wurden die verschiedenen Proben der pflanzenbasierten Pulver in eine Standardformulierung für einen veganen Pflanzenquark eingearbeitet und die Textur der resultierenden Produkte ermittelt. Die Ergebnisse befinden sich ebenfalls in **Tabelle 1,** letzte Spalte.

**Tabelle 1**

| Bsp. | Ursprung | pH* | Löslich-keit* | Onset [°C] | Endset [°C] | Peak [°C] | Reaktionsenthalpie [J/g] | Peakhöhe [mW] | Spezifische Oberfläche [cm$^2$/cm$^3$] |
|---|---|---|---|---|---|---|---|---|---|
| Datensatz für Qualitätsbestimmung | | | | | | | | | |
| 1 | Erbse | 7,77 | 0,4 | 56,275 | 70,7 | 63,46 | -0,585 | 0,02963 | 2029,9 |
| 2 | Erbse | 7,7 | 0,3 | 52,65 | 72,485 | 64,965 | -2,265 | 0,103005 | 2359,45 |
| 3 | Erbse | 7,77 | 0,325 | 52,825 | 70,12 | 62,64 | -1,305 | 0,05987 | 1386,25 |
| 4 | Erbse | 7,62 | 0,375 | 52,44 | 69,49 | 60,47 | -0,69 | 0,048685 | 1021,1 |
| 5 | Erbse | 7,98 | 0,4 | 53,27 | 71,08 | 62,725 | -1,095 | 0,069085 | 1316,45 |
| 6 | Erbse | 7,76 | 0,375 | 59,37 | 68,31 | 63,065 | -1,345 | 0,080835 | 1130,9 |
| 7 | Erbse | 7,39 | 0,2 | 55,54 | 66,67 | 61,425 | -0,775 | 0,09297 | 545,137705 |
| 8 | Erbse | 7,67 | 0,275 | 51,74 | 68,445 | 60,94 | -1,71 | 0,11 | 814,445 |
| 9 | Ackerbohne | 7,21 | 0,4 | 55,18 | 66,86 | 61,375 | -0,305 | 0,02612 | 1756 |
| 10 | Ackerbohne | 7,08 | 0,25 | 54,27 | 70,105 | 63,055 | -4,64 | 0,185 | 7011,15 |
| 11 | Kichererbse | 7,76 | 0,4 | 56,555 | 73,115 | 64,875 | -1,42 | 0,06824 | 1654,15 |
| 12 | Hafer | 7,74 | 0,3 | 53,505 | 69,175 | 62,065 | -0,97 | 0,04052 | 1371,2 |
| *) 1 Gew.-%ige Lösung | | | | | | | | | |

EP 4 553 497 B1

| Bsp. | Ursprung | D10 [μm] | D50 [μm] | D90 [μm] | FA** | WA*** | L* | a* | b* | Textur |
|---|---|---|---|---|---|---|---|---|---|---|
| 1 | Erbse | 16,41019 | 36,679675 | 73,50104 | 0,695 | 3,295 | 82,4 | 2,89 | 19,36 | strukturiert |
| 2 | Erbse | 16,04886 | 28,1959 | 50,241605 | 0,61 | 1,2 | 85,3 | 0,77 | 25,19 | nicht strukturiert |
| 3 | Erbse | 24,950615 | 49,37172 | 121,441215 | 0,66 | 2,775 | 80,68 | 2,8 | 18,17 | strukturiert |
| 4 | Erbse | 31,059785 | 72,287075 | 183,973495 | 1,005 | 2,49 | 82,32 | 4,24 | 21,51 | gummiartig |
| 5 | Erbse | 27,636675 | 51,3814 | 98,7448 | 0,45 | 2,46 | 79,85 | 3,67 | 19,15 | strukturiert |
| 6 | Erbse | 31,891985 | 59,106525 | 117,480105 | 0,63 | 2,34 | 81,41 | 3,49 | 18,9 | strukturiert |
| 7 | Erbse | 24,270895 | 98,484615 | 204,588875 | 0,785 | 1,155 | 77,18 | 6,08 | 19,02 | nicht strukturiert |
| 8 | Erbse | 36,621365 | 117,186405 | 222,080115 | 0,86 | 1,155 | 75,76 | 7,32 | 19,94 | nicht strukturiert |
| 9 | Ackerbohne | 19,44992 | 41,04005 | 84,19119 | 0,63 | 2,27 | 87,52 | 0,46 | 13,18 | mehlig |
| 10 | Ackerbohne | 5,51351 | 8,9755 | 15,646215 | 0,945 | 1,365 | 89,53 | 0,54 | 13,05 | nicht strukturiert |
| 11 | Kichererbse | 20,88119 | 42,092875 | 87,612445 | 0,68 | 2,91 | 83,79 | 1,42 | 16,25 | strukturiert |
| 12 | Hafer | 24,35504 | 51,666535 | 119,96077 | 0,79 | 1,505 | 78,96 | 3,01 | 17,21 | Stumpf, mehlig |

** Fettaufnahme [g Fett/g Pulver] *** Wasseraufnahme [g Wasser/g Pulver]

### Beispiel 1C

*Überprüfung des Zusammenhangs der Daten mittels PCA = Principal Component Analysis*

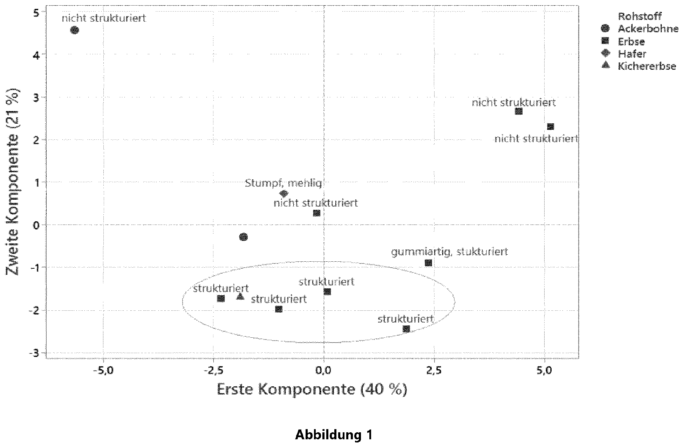

Der dritte Schritt des erfindungsgemäßen Verfahrens ist in **Abbildung 1** wiedergegeben. Dabei wird der zuvor gewonnene Datensatz mit Hilfe der Principal Component Analysis aufbereitet. Die PCA liefert ein Koordinatensystem, bei dem für jeden Datenpunkt die Scores für die ersten beiden Hauptkomponenten aufgetragen werden. In Abbildung 1 beträgt die erklärte Varianz der Hauptkomponente 1 40,8 %. Die der zweiten Hauptkomponente 26,1 %. Insgesamt erklärt die PCA 66,9 % der Varianz der Originaldaten. Die Kenntnis, welche Parameter die beiden Hauptkomponenten prägen, spielt für das Verfahren keine Rolle, da es in diesem Schritt nur darum geht, die Varianz auf eine skalierbare Größe zu bringen.

Wie man dem Diagramm entnehmen kann, verteilen sich die Datenpunkte mehr oder weniger gleichmäßig über das Koordinatensystem. Nach Zuordnung der tatsächlich gefundenen Texturen zu den Datenpunkten zeigt sich jedoch, dass das Diagramm Cluster gleicher oder ähnlicher Struktur enthält. So finden sich die erwünschten strukturierten Texturen ausschließlich im x-Koordinatenbereich -2,5 bis 2,5 und y-Koordinatenbereich +1 bis +3. Proben, die sich nicht in diesem Cluster befinden, waren nicht strukturiert, hier reichte die Textur von mehlig, cremig bis gummiartig.

Abbildung 1 kann daher als Rohstoff-Auswahldiagramm verstanden werden. Um die zu erwartenden funktionellen Eigenschaften eines Rohstoffs vorhersagen zu können, bedarf es lediglich der Erfassung der physikochemischen Daten entsprechend den gewählten Prüfparametern - die in der Regel ohnehin entweder durch die Spezifikation vorgegeben sind oder bei der Eingangskontrolle erfasst werden - und der Berechnung des entsprechenden Datenpunktes wie oben beschrieben. Aus der Lage des Datenpunktes im Diagramm kann dann auf die zu erwartende Rohstofffunktionalität im Endprodukt geschlossen werden.

### Beispiel 2

Nachfolgend wird das erfindungsgemäße Verfahren an Hand der Beurteilung von 17 verschiedenen Milchproteinpulvern erläutert;

Aus den zur Verfügung stehenden Parametern wurden die folgenden ausgewählt:

- pH-Wert
- Löslichkeit

- DSC-Daten (Onset, Endset, Peak)
- Partikelgrößenverteilung (10, D50, D90, spezifische Oberfläche)
- Fettaufnahmevermögen (Rapsöl)
- Wasseraufnahmevermögen
- Farbkoordinaten (L*, a*, b*)

und die entsprechenden Messwerte bestimmt; diese sind in **Tabelle 2** zusammengefasst.

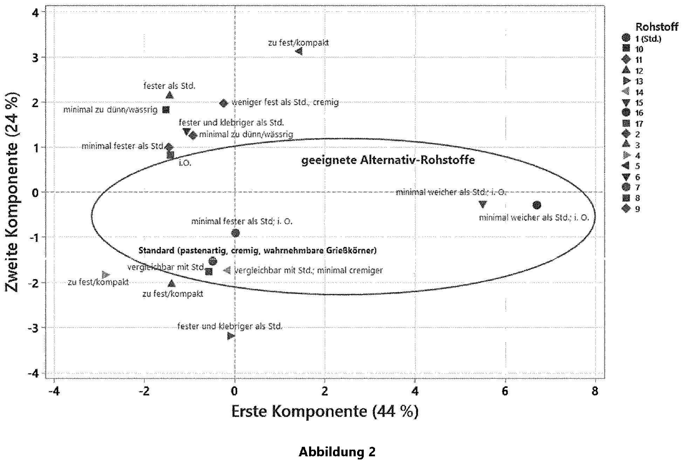

Anschließend wurden die verschiedenen Proben der Milchproteinpulver in eine Standardformulierung für Milchgrieß mit hohem Proteingehalt eingearbeitet und die Textur der resultierenden Produkte ermittelt. Die Ergebnisse befinden sich ebenfalls in Tabelle 2, letzte Spalte. Anschließend wurde eine PCA entsprechend Beispiel 1C durchgeführt. Der Score Plot mit dem Cluster an Stoffen mit gewünschten Eigenschaften findet sich in **Abbildung 2.**

**Tabelle 2**

| Datensatz für Qualitätsbestimmung | | | | | | |
|---|---|---|---|---|---|---|
| Bsp. | pH* | Löslichkeit* | Onset [°C] | Endset [°C] | Peak [°C] | Spezifische Oberfläche [$cm^2/cm^3$] |
| 1 | 7,78 | 0,30 | 0,00 | 0,00 | 0,00 | 7,78 |
| 2 | 7,85 | 0,48 | 60,93 | 72,87 | 64,35 | 7,85 |
| 3 | 7,38 | 0,65 | 0,00 | 0,00 | 0,00 | 7,38 |
| 4 | 7,83 | 0,65 | 0,00 | 0,00 | 0,00 | 7,83 |
| 5 | 7,84 | 0,40 | 61,21 | 66,48 | 64,03 | 7,84 |
| 6 | 7,85 | 0,40 | 52,84 | 59,18 | 55,63 | 7,85 |
| 7 | 7,60 | 0,50 | 0,00 | 0,00 | 0,00 | 7,60 |
| 8 | 7,68 | 0,60 | 0,00 | 0,00 | 0,00 | 7,68 |
| 9 | 7,61 | 0,50 | 61,51 | 67,92 | 67,92 | 7,61 |
| 10 | 7,75 | 0,63 | 61,35 | 77,76 | 66,28 | 7,75 |
| 11 | 7,47 | 0,40 | 56,88 | 72,93 | 66,29 | 7,47 |
| 12 | 7,88 | 0,65 | 62,19 | 71,66 | 66,83 | 7,88 |
| 13 | 7,57 | 0,30 | 0,00 | 0,00 | 0,00 | 7,57 |
| 14 | 7,82 | 0,40 | 0,00 | 0,00 | 0,00 | 7,82 |
| 15 | 7,73 | 0,05 | 0,00 | 0,00 | 0,00 | 7,73 |
| 16 | 7,74 | 0,01 | 0,00 | 0,00 | 0,00 | 7,74 |
| 17 | 7,91 | 0,15 | 58,15 | 61,80 | 60,36 | 7,91 |

| Bsp. | D10 [μm] | D50 [μm] | D90 [μm] | FA** | WA*** | L* | a* | b* | Textur |
|---|---|---|---|---|---|---|---|---|---|
| 1 | 23,98 | 55,87 | 167,72 | 2,23 | 1,37 | 93,34 | -1,21 | 9,14 | pastenartig, cremig, wahrnehmbare Grießkörner |
| 2 | 35,52 | 86,49 | 222,32 | 2,36 | 3,64 | 91,91 | -0,74 | 9,92 | weniger fest als Std., cremig |
| 3 | 23,04 | 61,05 | 168,18 | 2,23 | 4,42 | 92,75 | -1,14 | 9,88 | zu fest/kompakt |
| 4 | 17,70 | 41,80 | 88,29 | 2,30 | 4,65 | 94,18 | -1,71 | 9,42 | vergleichbar mit Std. |
| 5 | 31,96 | 171,26 | 322,69 | 1,31 | 3,62 | 92,13 | -1,34 | 12,29 | zu fest/kompakt |
| 6 | 27,75 | 69,27 | 186,03 | 2,08 | 3,22 | 92,54 | -1,26 | 9,35 | fester und klebriger als Std. |

(fortgesetzt)

| Bsp. | D10 [$\mu$m] | D50 [$\mu$m] | D90 [$\mu$m] | FA** | WA*** | L* | a* | b* | Textur |
|---|---|---|---|---|---|---|---|---|---|
| 7 | 26,17 | 93,50 | 250,93 | 1,73 | 3,73 | 92,75 | -1,14 | 9,88 | minimal fester als Std; in Ordnung |
| 8 | 25,48 | 62,62 | 193,51 | 2,42 | 4,53 | 92,12 | -1,36 | 12,65 | minimal zu dünn/wässrig |
| 9 | 33,96 | 74,92 | 225,91 | 2,60 | 3,55 | 92,98 | -1,80 | 10,75 | minimal zu dünn/wässrig |
| 10 | 27,10 | 69,60 | 197,09 | 2,36 | 3,89 | 92,47 | -0,28 | 7,77 | minimal zu dünn/wässrig |
| 11 | 21,24 | 64,05 | 190,68 | 2,39 | 3,98 | 92,50 | -0,69 | 9,35 | minimal fester als Std. |
| 12 | 28,66 | 71,08 | 207,07 | 1,58 | 3,19 | 93,18 | -2,12 | 11,92 | fester als Std. |
| 131 | 25,64 | 53,25 | 102,00 | 3,59 | 2,25 | 90,93 | -1,65 | 12,86 | fester und klebriger als Std. |
| 14 | 28,19 | 65,22 | 185,75 | 2,62 | 3,19 | 92,29 | -1,84 | 12,10 | vergleichbar mit Std., minimal cremiger |
| 15 | 42,96 | 175,53 | 368,09 | 2,09 | 0,30 | 88,26 | -0,57 | 13,74 | minimal weicher als Std.; in Ordnung |
| 16 | 58,08 | 187,30 | 392,03 | 2,64 | 0,14 | 87,71 | -0,37 | 13,87 | minimal weicher als Std.; in Ordnung |
| 17 | 18,25 | 50,40 | 111,51 | 2,14 | 1,15 | 93,39 | -2,34 | 12,61 | In Ordnung |
| * 1%ige Lösung ** Fettaufnahme [g Fett/g Pulver] *** Wasseraufnahme [g Wasser/g Pulver] | | | | | | | | | |

**Patentansprüche**

1. Verfahren zur Vorhersage der Rohstofffunktionalität in Endprodukten wie Milchprodukten, Produkten auf primärer und hauptsächlicher Basis von Milch inklusive von Käsen deren pflanzlichen Milchalternativen sowie entsprechenden Hybridprodukten, umfassend oder bestehend aus den folgenden Schritten

   (a) Bereitstellen eines Satzes von Rohstoffen unterschiedlicher Qualitäten;
   (b) Erstellen eines Sets von Prüfparametern;
   (c) Anwendung der festgelegten Prüfparameter auf jede der einzelnen Proben aus Schritt (a) unter Erzeugung eines Datensatzes;
   (d) Einarbeiten der Rohstoffe unterschiedlicher Qualitäten aus Schritt (a) in Endprodukte, wie Milchprodukte, Produkte auf primärer und hauptsächlicher Basis von Milch inklusive von Käsen deren pflanzlichen Milchalternativen sowie entsprechenden Hybridprodukten;
   (e) Erfassung und Bewertung der Produkteigenschaften;
   (f) Überprüfung des Zusammenhangs der Daten im Datensatz mit Hilfe der Principal Component Analysis (PCA) unter Erhalt eines Koordinatensystems (Score-Plot) und Ermittlung der größten Varianz der Daten als erster Komponente und der zweitgrößten Varianz der Daten als zweiter Komponente;
   (g) Zuordnung der gefundenen Produkteigenschaften aus Schritt (e) zu den Datenpunkten im Koordinatensystem aus Schritt (f); und
   (h) Ermitteln von Clustern im Score-Plot zur Identifikation von Rohstoffen, die beim Einsatz im Endprodukt zu den gewünschten ähnlichen oder gleichen Produkteigenschaften führen.

2. Verfahren nach Anspruch 1, **dadurch gekennzeichnet, dass** man als Endprodukte Milchprodukte, Produkte auf primärer und hauptsächlicher Basis von Milch inklusive von Käsen deren pflanzlichen Milchalternativen sowie entsprechenden Hybridprodukten einsetzt, die ausgewählt sind aus der Gruppe, die gebildet wird von Vollmilch, Magermilch, UHT-Milch, Milchpulvern, Sahne, Quark, Käse und Joghurt, und Käse sowie deren pflanzlichen Ersatzprodukten und Applikationen wie u.a. pflanzliche Drinks, Spreads, Sahnealternativen, Joghurtalternativen und Käsealternativen.

3. Verfahren nach Anspruch 2, **dadurch gekennzeichnet, dass** man milch- und/oder pflanzenbasierte Rohstoffe/Inhaltsstoffen/Zutaten einsetzt, die ausgewählt sind aus der Gruppe, die gebildet wird von Proteinen, Fetten, Kohlenhydraten, Sauermolke, Milchpermeaten und Milchretentaten sowie deren Mischungen und Konzentraten

4. Verfahren nach mindestens einem der Ansprüche 1 bis 3, **dadurch gekennzeichnet, dass** man einen Rohstoffprobensatz einsetzt, der 5 bis 50 Proben umfasst.

5. Verfahren nach mindestens einem der Ansprüche 1 bis 4, **dadurch gekennzeichnet, dass** man einen Probensatz einsetzt, der Rohstoffe von mindestens drei verschiedenen Qualitäten umfasst.

6. Verfahren nach mindestens einem der Ansprüche 1 bis 5, **dadurch gekennzeichnet, dass** man ein Set von Prüfparametern einsetzt, das mindestens zwei der folgenden Parameter umfasst:

   - pH-Wert;
   - Löslichkeit;
   - thermisches Verhalten (DSC);
   - der Pulver;
   - Partikelgrößenverteilung (D10, D50, D90, spezifische Oberfläche);
   - Fettaufnahmevermögen (Rapsöl);
   - Wasseraufnahmevermögen;
   - gelöste Sauerstoffmenge;
   - Instabilitätsindex (Lumisizer);
   - Helligkeitsfarbwert weiß/schwarz (L*);
   - Farbkoordinate rot/grün (a*);
   - Farbkoordinate gelb/blau (b*);
   - freier Aminostickstoff (PAN);
   - Ammoniakgehalt;
   - Harnstoffgehalt;
   - Calciumgehalt;
   - Säuregehalt;
   - rheologisches Verhalten;
   - Festigkeit; sowie
   - Syneräsegrad.

7. Verfahren nach Anspruch 6, **dadurch gekennzeichnet, dass** man ein Set von Prüfparametern einsetzt, das mindestens 5, vorzugsweise mindestens 7 Parameter umfasst.

8. Verfahren nach mindestens einem der Ansprüche 1 bis 7, **dadurch gekennzeichnet, dass** man Produkteigenschaften vorhersagt, die ausgewählt sind aus der Gruppe, die gebildet wird von Textur, Cremigkeit, Synärese, Mundgefühl, Festigkeit, Emulsionsstabilität, Synergieeffekte sowie Kombinationen von zwei, drei oder mehreren dieser Eigenschaften und Vorhersage ihrer Applikationseigenschaften.

**Claims**

1. A method for predicting raw material functionality in end products such as dairy products, primary and main milk-based products including cheese, their plant-based milk alternatives and corresponding hybrid products, comprising or consisting of the following steps

   (a) providing a set of raw materials of different qualities;
   (b) establishing a set of test parameters;
   (c) applying the set of test parameters to each of the individual samples from step (a) to generate a data set;
   (d) incorporating the raw materials of different qualities from step (a) into final products, such as dairy products, products based primarily and principally on milk, including cheeses whose plant-based milk alternatives and corresponding hybrid products;

(e) recording and evaluation of product characteristics;

(f) checking the correlation of the data in the data set using Principal Component Analysis (PCA) to obtain a coordinate system (score plot) and determining the largest variance of the data as the first component and the second largest variance of the data as the second component;

(g) assigning the product properties found from step (e) to the data points in the coordinate system from step (f); and

(h) determining clusters in the score plot to identify raw materials which, when used in the end product, lead to the desired similar or identical product properties.

2. The method of Claim 1, wherein the end products used are milk products, products based primarily and mainly on milk, including cheeses, their plant-based milk alternatives and corresponding hybrid products, which are selected from the group formed by whole milk, skimmed milk, UHT milk, milk powders, cream, curd, cheese and yoghurt, and cheese and their plant-based substitute products and applications such as, among others including plant-based drinks, spreads, cream alternatives, yoghurt alternatives and cheese alternatives.

3. The method of Claim 2, wherein milk- and/or plant-based raw materials/ingredients/ingredients are used which are selected from the group formed by proteins, fats, carbohydrates, acid whey, milk permeates and milk retentates as well as mixtures and concentrates thereof

4. The method of Claim 1, wherein raw material sample set comprising 5 to 50 samples is used.

5. The method of Claim 1, wherein sample set is used which comprises raw materials of at least three different qualities.

6. The method of Claim 1, wherein a set of test parameters is used which comprises at least two of the following parameters:

- pH value;
- solubility;
- thermal behavior (DSC) of the powder;
- particle size distribution (D10, D50, D90, specific surface area);
- fat absorption capacity (rapeseed oil);
- water absorption capacity;
- dissolved oxygen quantity;
- instability index (Lumisizer);
- lightness color value white/black ($L^*$);
- color coordinate red/green ($a^*$);
- color coordinate yellow/blue ($b^*$);
- free amino nitrogen (PAN);
- ammonia content;
- urea content;
- calcium content;
- acidity;
- rheological behavior;
- firmness; and
- degree of syneresis.

7. The method of Claim 6, wherein a set of test parameters is used which comprises at least 5, preferably at least 7 parameters.

8. The method of Claim 1, wherein properties are predicted which are selected from the group formed by texture, creaminess, syneresis, mouthfeel, firmness, emulsion stability, synergy effects as well as combinations of two, three or more of these properties and prediction of their application properties.

**Revendications**

1. Procédé permettant de prédire la fonctionnalité des matières premières dans les produits finis tels que les produits laitiers, les produits primaires et principaux à base de lait, y compris le fromage, leurs substituts végétaux et les produits hybrides correspondants, comprenant ou consistant en les étapes suivantes :

(a) fournir un ensemble de matières premières de différentes qualités ;

(b) établir un ensemble de paramètres de test ;

(c) appliquer l'ensemble de paramètres de test à chacun des échantillons individuels de l'étape (a) afin de générer un ensemble de données ;

(d) incorporer les matières premières de différentes qualités de l'étape (a) dans des produits finaux, tels que des produits laitiers, des produits à base principalement et essentiellement de lait, y compris des fromages, leurs substituts végétaux et les produits hybrides correspondants ;

(e) enregistrer et évaluer les caractéristiques des produits ;

(f) vérifier la corrélation des données dans l'ensemble de données à l'aide d'une analyse en composantes principales (ACP) afin d'obtenir un système de coordonnées (graphique de scores) et déterminer la plus grande variance des données comme première composante et la deuxième plus grande variance des données comme deuxième composante ;

(g) attribuer les propriétés du produit trouvées à l'étape (e) aux points de données dans le système de coordonnées de l'étape (f) ; et

(h) déterminer des grappes dans le graphique de score afin d'identifier les matières premières qui, lorsqu'elles sont utilisées dans le produit final, conduisent aux propriétés souhaitées similaires ou identiques du produit.

2. Procédé selon la revendication 1, dans lequel les produits finaux utilisés sont des produits laitiers, des produits à base principalement et essentiellement de lait, y compris les fromages, leurs substituts végétaux et les produits hybrides correspondants, qui sont sélectionnés dans le groupe formé par le lait entier, le lait écrémé, le lait UHT, les poudres de lait, la crème, le caillé, le fromage et le yaourt, ainsi que les fromages et leurs produits de substitution à base végétale et leurs applications telles que, entre autres, les boissons à base végétale, les pâtes à tartiner, les substituts de crème, les substituts de yaourt et les substituts de fromage.

3. Procédé selon la revendication 2, dans lequel on utilise des matières premières/ingrédients/ingrédients à base de lait et/ou végétaux qui sont choisis parmi le groupe formé par les protéines, les graisses, les glucides, le lactosérum acide, les perméats de lait et les rétentats de lait, ainsi que leurs mélanges et concentrés.

4. Procédé selon la revendication 1, dans lequel on utilise un ensemble d'échantillons de matières premières comprenant 5 à 50 échantillons.

5. Procédé selon la revendication 1, dans lequel on utilise un ensemble d'échantillons qui comprend des matières premières d'au moins trois qualités différentes.

6. Procédé selon la revendication 1, dans lequel on utilise un ensemble de paramètres d'essai qui comprend au moins deux des paramètres suivants :

- valeur du pH ;
- solubilité ;
- comportement thermique (DSC) de la poudre ;
- distribution granulométrique (D10, D50, D90, surface spécifique) ;
- capacité d'absorption des graisses (huile de colza) ;
- capacité d'absorption de l'eau ;
- quantité d'oxygène dissous ;
- indice d'instabilité (Lumisizer) ;
- valeur de luminosité blanche/noire (L*) ;
- coordonnée de couleur rouge/verte (a*) ;
- coordonnée de couleur jaune/bleue (b*) ;
- azote aminé libre (PAN) ;
- teneur en ammoniac ;
- teneur en urée ;
- teneur en calcium ;
- acidité ;
- comportement rhéologique ;
- fermeté ; et
- degré de synérèse.

7. Procédé selon la revendication 6, dans lequel on utilise un ensemble de paramètres d'essai qui comprend au moins 5, de préférence au moins 7 paramètres.

8. Procédé selon la revendication 1, dans lequel les propriétés sont prédites parmi celles sélectionnées dans le groupe formé par la texture, l'onctuosité, la synérèse, la sensation en bouche, la fermeté, la stabilité de l'émulsion, les effets de synergie, ainsi que les combinaisons de deux, trois ou plusieurs de ces propriétés et la prédiction de leurs propriétés d'application.

**Abbildung 1**

**Abbildung 2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1626275 B1
- EP 2378354 B1
- EP 2581785 B1
- WO 2009056591 A1
- EP 3875953 B1
- JP 2022121218 B

**In der Beschreibung aufgeführte Nicht-Patentliteratur**

- **MÜLLER, K**. Sauerstoff-Durchlässigkeit von Kunststoffflaschen und Verschlüssen''. *Dissertation TU München*, 2003